# EUROPEAN PATENT SPECIFICATION

(11) **EP 3 076 811 B1**
(45) Date of publication and mention of the grant of the patent: **07.08.2019**
(21) Application number: 14806657.4
(22) Date of filing: 04.12.2014
(51) Int. Cl.: A24F 47/00

(54) **HEATED AEROSOL GENERATING ARTICLE WITH THERMAL SPREADING ENDPIECE**
BEHEIZTER AEROSOL-ERZEUGENDER ARTIKEL MIT WÄRMESTREUENDEM ENDSTÜCK
ARTICLE DE GÉNÉRATION D'AÉROSOL CHAUFFÉ AVEC UN EMBOUT D'ÉTALEMENT THERMIQUE

(30) Priority: 05.12.2013 EP 13195907
(43) Date of publication of application: 12.10.2016
(73) Proprietor: Philip Morris Products S.A., 2000 Neuchâtel (CH)
(72) Inventor: MALGAT, Alexandre, 1422 Les Tuileries de Grandson (CH); ROUDIER, Stephane, 2013 Colombier (CH); BORGES COURAÇA, Ana Carolina, 1005 Lausanne (CH); LAVANCHY, Frederic, 1373 Chavornay (CH); MEYER, Cedric, 1006 Lausanne (CH)
(74) Representative: Bates, Alan Douglas Henry
(86) International application number: PCT/EP2014/076652
(87) International publication number: WO 2015/082654

(56) References cited:
- EP-A1- 2 609 821
- EP-A2- 0 277 519
- WO-A2-2013/102609
- US-A1- 2010 006 113
- US-A1- 2013 056 013

## Description

The present specification relates to heated aerosol-generating articles for use with an aerosol-generating device comprising a heating element, the articles having a lowered propensity for ignition, for example when brought into contact with a flame.

Aerosol-generating articles in which an aerosol-forming substrate, such as a tobacco containing substrate, is heated rather than combusted are known in the art. The aim of such heated aerosol-generating articles is to reduce known harmful smoke constituents produced by the combustion and pyrolytic degradation of tobacco in conventional cigarettes. A conventional cigarette is lit when a user applies a flame to one end of the cigarette and draws air through the other end. The localised heat provided by the flame and the oxygen in the air drawn through the cigarette cause the end of the cigarette to ignite, and the resulting combustion generates an inhalable smoke. By contrast in heated aerosol-generating articles, an inhalable aerosol is typically generated by the transfer of heat from a heat source to a physically separate aerosol-forming substrate or material, which may be located within, around or downstream of the heat source. During consumption, volatile compounds are released from the aerosol-forming substrate by heat transfer from the heat source and entrained in air drawn through the aerosol-generating article. As the released compounds cool, they condense to form an aerosol that is inhaled by the consumer.

Heated aerosol-generating articles comprising tobacco for generation of an aerosol by heating rather than burning are known in the art. For example, WO2013/102614 discloses an aerosol-generating system comprising a heated aerosol-generating article and an aerosol-generating device having a heater for heating the heated aerosol-generating article to produce an aerosol. WO 2013/102609 A discloses an aerosol-generating article in from of a rod having a mouth end, a distal end and an aerosol-generating article in-between.

Tobacco used as part of an aerosol-forming substrate in heated aerosol-generating articles is designed to produce an aerosol when heated rather than when burned. Thus, such tobacco typically contains high levels of aerosol formers, such as glycerine or propylene glycol. If a user were to light a heated aerosol-generating article and smoke it as if it were a conventional cigarette that user would not receive the intended user experience. It would be desirable to produce a heated aerosol-generating article that has a lowered propensity for flame ignition. Such a heated aerosol-generating article would be preferably difficult to light during attempts to light the article with a lighter, such as a flame, in the manner of traditional cigarettes.

A heated aerosol-generating article is provided in the form of a rod having a mouth end and a distal end upstream from the mouth end. The aerosol-generating article has an aerosol-forming substrate located upstream of the mouth end within the rod and the distal end of the heated aerosol-generating article is spanned by a non-flammable thermally-conductive material. The heated aerosol-generating article is for use with an aerosol-generating device comprising a heating element. If a heat source, such as a flame or other cigarette lighter, is applied to the distal end of the heated aerosol-generating article, the thermally-conductive material that spans the distal end of the aerosol-generating article conducts a portion of the heat away from the point of contact with the heat source. Thus, more thermal energy needs to be supplied in order to raise the temperature of the aerosol-forming substrate within the heated aerosol-generating article to its ignition point. This reduces the propensity for ignition of the aerosol-forming substrate. Thus, the thermally-conductive material acts as a thermally-conducting flame barrier for preventing heat from being drawn into the aerosol-generating article and mitigating against the risk of a user inadvertently igniting the aerosol-forming substrate by applying a flame, or other ignition source, to the aerosol-generating article.

Preferably, the aerosol-generating article is a smoking article that generates an aerosol that is directly inhalable into a user's lungs through the user's mouth. More, preferably, the aerosol-generating article is a smoking article that generates a nicotine-containing aerosol that is directly inhalable into a user's lungs through the user's mouth.

As used herein, the term 'aerosol-generating device' is used to describe a device that interacts with an aerosol-forming substrate of an aerosol-generating article to generate an aerosol. Preferably, the aerosol-generating device is a smoking device that interacts with an aerosol-forming substrate of an aerosol-generating article to generate an aerosol that is directly inhalable into a user's lungs thorough the user's mouth. The aerosol-generating device may be a holder for a smoking article.

For the avoidance of doubt, the term 'heating element' is used to mean one or more heating elements.

The thermally-conductive material is a non-flammable material. The thermally-conductive material is preferably a metal foil, such as aluminium foil. The thermally-conductive material may comprise a metal foil, such as aluminium foil. For example, the thermally-conductive material may be a co-laminated sheet comprising aluminium foil and a second material such as paper. Aluminium foil is a highly efficient thermal conductor either on its own or as a layer in a co-laminated sheet. The thermally-conductive material is not a carbonaceous material.

The heated aerosol-generating article may comprise a plurality of elements, including the aerosol-forming substrate, assembled within a wrapper, such as a cigarette paper, to form a rod. The thermally-conductive material is arranged to span the distal end of the rod and help prevent the influx of heat into the rod. The thermally-conductive material spreads heat away from the distal end of the heated aerosol-generating device to lower the risk of igniting the aerosol-forming substrate.

The thermally-conductive material spans the distal end of the rod and may extend downstream along the rod to dissipate heat directly applied to the distal end of the rod, thereby reducing the propensity for ignition of the rod. For example the thermally-conductive material may extend by a distance of at least 5 mm downstream along the rod, preferably at least 10 mm downstream along the rod.

The heated aerosol-generating article may be in the form of a rod having a mouth end and a distal end upstream from the mouth end, in which a spacer element is located within the rod upstream of the aerosol-forming substrate. The spacer element may be in the form of a tube. The thermally-conductive material may be located by the spacer element. For example, the spacer element may be in the form of a tube and the thermally-conductive material may span an end of the tube.

The thermally-conductive material spans the distal end of the aerosol-generating article such that flow of air into the distal end of the article is prevented. The thermally-conductive material is rupturable to enable air flow into the distal end.

In preferred embodiments of a heated aerosol-forming article, the aerosol-forming substrate may comprise a gathered sheet of aerosol-forming material circumscribed by a wrapper. The gathered sheet of aerosol-forming material may be a sheet of tobacco such as a sheet of homogenised tobacco.

The aerosol-forming substrate may be formed as a rod of cut filler, and the rod of cut filler may be encircled by a wrapper.

The heated aerosol-generating article is preferably for use with an aerosol-generating device that comprises an insertable heating element for insertion into a distal end of the heated aerosol-generating article. The heating element may be brought into contact with the aerosol-forming substrate within the aerosol-generating article by removing the thermally-conductive material or by piercing the thermally-conductive material. Prior to use, the thermally-conductive material provides some mitigation against ignition of the aerosol-forming substrate using an external ignition source such as a flame.

The aerosol-forming substrate may be in the form of a rod comprising aerosol-forming material. A rod may be provided comprising a gathered sheet of aerosol-forming material circumscribed by a wrapper. Such a rod may be assembled within a cigarette paper, or other suitable material, as an aerosol-forming substrate of an aerosol-generating article.

Preferably the sheet of aerosol-forming material comprises tobacco, for example tobacco that may be classed as homogenised, reconstituted or cast leaf tobacco.

The gathered sheet of material preferably extends along substantially the entire rod length of the rod and across substantially the entire transverse cross-sectional area of the rod.

Preferably, rods according to the specification are of substantially uniform cross-section.

Rods according to various aspects of the specification may be produced having different dimensions depending upon their intended use. The heated aerosol-generating article is in the form of a rod and the aerosol-forming substrate, which is a component part of the heated aerosol-generating article, may also be in the form of a rod.

Rods according to the specification may have a diameter of between about 5 mm and about 10 mm depending upon their intended use.

For example, rods according to the specification may have a rod length of between about 5 mm and about 150 mm depending upon their intended use.

In preferred embodiments, rods according to the specification for use as aerosol-forming substrates in heated aerosol-generating articles may have a rod length of between about 5 mm and about 20 mm or about 30 mm.

Rods according to the specification of a desired unit rod length may be produced by forming a rod of multiple unit rod length and then cutting or otherwise dividing the rod of multiple unit rod length into multiple rods of the desired unit rod length.

For example, rods having a rod length of about 15 mm for use as aerosol-forming substrates in heated aerosol-generating articles may be produced by forming a rod having a rod length of about 150 mm and then severing the elongate rod into ten rods having a rod length of about 15 mm.

As used herein, the term 'rod' is used to denote a generally cylindrical element of substantially circular, oval or elliptical cross-section.

As used herein, the term 'sheet' denotes a laminar element having a width and length substantially greater than the thickness thereof. The width of a sheet is greater than 10 mm, preferably greater than 20 mm or 30 mm.

As used herein, the term "co-laminated sheet" denotes a single sheet formed from two or more layers of material in intimate contact with one another.

As used herein, the term "aerosol-forming material" denotes a material that is capable of releasing volatile compounds upon heating to generate an aerosol. An aerosol-forming substrate may comprise or consist of an aerosol-forming material.

As used herein, the term 'rod length' denotes the dimension in the direction of the cylindrical axis of rods as described herein.

As used herein, the term 'homogenised tobacco material' denotes a material formed by agglomerating particulate tobacco.

As used herein, the term 'gathered' denotes that the sheet of tobacco material is convoluted, folded, or otherwise compressed or constricted substantially transversely to the cylindrical axis of the rod.

As used herein, the terms 'upstream' and 'downstream' are used to describe the relative positions of components, or portions of components, of aerosol-generating articles comprising rods as described herein in relation to the direction of air drawn through the aerosol-generating articles during use thereof.

The gathered sheet of aerosol-forming material may be a textured sheet of material. Use of a textured sheet of material may advantageously facilitate gathering of the sheet to form an aerosol-forming substrate as described herein.

As used herein, the term 'textured sheet' denotes a sheet that has been crimped, embossed, debossed, perforated or otherwise deformed. Textured sheets of material may comprise a plurality of spaced-apart indentations, protrusions, perforations or a combination thereof.

As used herein, the term 'crimped sheet' is intended to be synonymous with the term 'creped sheet' and denotes a sheet having a plurality of substantially parallel ridges or corrugations.

A number of aerosol-generating articles in which an aerosol-forming substrate is heated rather than combusted have been proposed in the art. Typically in heated aerosol-generating articles, an aerosol is generated by the transfer of heat from a heat source, for example a chemical, electrical or combustible heat source, to a physically separate aerosol-forming substrate, which may be located within, around or downstream of the heat source.

As used herein, the term 'aerosol-forming substrate' denotes a substrate consisting of or comprising an aerosol-forming material that is capable of releasing volatile compounds upon heating to generate an aerosol.

Rods used as aerosol-forming substrates in heated aerosol-generating articles are typically significantly shorter in rod length than rods of combustible smokable material in conventional lit-end smoking articles.

In preferred embodiments, the heated aerosol-generating articles described herein are for use in electrically-operated aerosol-generating systems in which the aerosol-generating substrate of the heated aerosol-generating article is heated by an electrical heat source. Such heated aerosol-generating articles are frequently constructed having an aerosol-forming substrate at a distal end. Thus, a user may inadvertently attempt to light the article in a traditional manner. The reduced ignition propensity of heated aerosol-generating articles in which the distal end of the aerosol-generating article is spanned by a thermally-conductive material may advantageously dissuade a user from attempting to ignite the article.

Heated aerosol-generating articles may be of the type disclosed in EP-A-0 822 670.

A system may be provided comprising an electrically-operated aerosol-generating apparatus and an aerosol-generating article for use with the apparatus. The aerosol-generating article is any heated aerosol-generating article as described herein.

Preferred embodiments of aerosol-generating articles comprise gathered sheets of homogenised tobacco material as the aerosol-forming substrate. In certain embodiments, sheets of homogenised tobacco material may have a tobacco content of at least about 40% by weight on a dry weight basis or of at least about 50% by weight on a dry weight basis. In other embodiments, sheets of homogenised tobacco material may have a tobacco content of about 70% or more by weight on a dry weight basis. The use of sheets of homogenised tobacco material having high tobacco content advantageously generates aerosols with enhanced tobacco flavour.

Sheets of homogenised tobacco material may comprise one or more intrinsic binders, that is tobacco endogenous binders, one or more extrinsic binders, that is tobacco exogenous binders, or a combination thereof to help agglomerate the particulate tobacco. Alternatively, or in addition, sheets of homogenised tobacco material may comprise other additives including, but not limited to, tobacco and non-tobacco fibres, aerosol-formers, humectants, plasticisers, flavourants, fillers, aqueous and non-aqueous solvents and combinations thereof.

Suitable extrinsic binders for inclusion in sheets of homogenised tobacco material are known in the art and include, but are not limited to: gums such as, for example, guar gum, xanthan gum, arabic gum and locust bean gum; cellulosic binders such as, for example, hydroxypropyl cellulose, carboxymethyl cellulose, hydroxyethyl cellulose, methyl cellulose and ethyl cellulose; polysaccharides such as, for example, starches, organic acids, such as alginic acid, conjugate base salts of organic acids, such as sodium-alginate, agar and pectins; and combinations thereof.

Homogenised tobacco material may comprise between about 1% and about 5% non-tobacco fibres by weight on a dry weight basis.

Suitable aerosol-formers and humectants for inclusion in sheets of homogenised tobacco material are known in the art and include, but are not limited to: polyhydric alcohols, such as triethylene glycol, 1,3-butanediol and glycerine; esters of polyhydric alcohols, such as glycerol mono-, di- or triacetate; and aliphatic esters of mono-, di- or polycarboxylic acids, such as dimethyl dodecanedioate and dimethyl tetradecanedioate.

For example, sheets of homogenised tobacco material may have an aerosol former content of between about 5% and about 30% by weight on a dry weight basis. Heated aerosol-generating articles may preferably include homogenised tobacco having an aerosol former content of greater than 5% to about 30%. The aerosol former may preferably be glycerine.

Sheets of homogenised tobacco material for use in forming heated aerosol-generating articles as described herein are preferably formed by a casting process of the type generally comprising casting a slurry comprising particulate tobacco and one or more binders onto a conveyor belt or other support surface, drying the cast slurry to form a sheet of homogenised tobacco material and removing the sheet of homogenised tobacco material from the support surface.

For example, in certain embodiments sheets of homogenised tobacco material may be formed from slurry comprising particulate tobacco, guar gum, cellulose fibres and glycerine by a casting process.

Sheets of homogenised tobacco material may be textured using suitable known machinery for texturing filter tow, paper and other materials.

For example, sheets of homogenised tobacco material may be crimped using a crimping unit of the type described in CH-A-691156, which comprises a pair of rotatable crimping rollers. However, it will be appreciated that sheets of homogenised tobacco material may be textured using other suitable machinery and processes that deform or perforate the sheets of homogenised tobacco material.

Preferably, sheets of tobacco material for use in forming aerosol-forming substrates of heated aerosol-generating articles have a width of at least about 25 mm. In certain embodiments sheets of material may have a width of between about 25 mm and about 300 mm. Preferably, the sheets of material have a thickness of at least about 50 µm to about 300 µm.

In certain embodiments, individual sheets of material may have a thickness of between 10 µm and about 250 µm. In certain embodiments, sheets of homogenised tobacco material may have a grammage 100 g/m² and about 300 g/m².

A method may be provided of forming an aerosol-forming substrate for a heated aerosol-generating article. The method may comprise the steps of: providing a continuous sheet comprising an aerosol-forming material; gathering the sheet transversely relative to the longitudinal axes thereof; circumscribing the gathered sheet with a wrapper to form a continuous rod, and severing the continuous rod into a plurality of discrete rods of aerosol-forming substrate. The aerosol-forming material may be any aerosol-forming material described above, and is preferably homogenised tobacco. In certain embodiments the wrapper is any suitable material such as a cigarette paper.

The method may further comprise texturing the continuous sheet. For example, the method may comprise crimping, embossing, perforating or otherwise texturing the continuous sheet prior to gathering.

Specific embodiments will be further described, by way of example only, with reference to the accompanying drawings in which:
Figure 1 shows a schematic cross-section of apparatus for forming a rod of aerosol-forming substrate for use in a heated aerosol-generating article;
Figures 2 illustrates an embodiment of an aerosol-generating article as described herein;
Figure 3 illustrates an alternative embodiment of an aerosol-generating article as described herein;
Figure 4 illustrates an aerosol-generating system comprising an electrically-operated aerosol-generating device and an aerosol-generating article as illustrated in Figure 2;
Figure 5 is a schematic cross-sectional diagram of the aerosol-generating device illustrated in Figure 4.

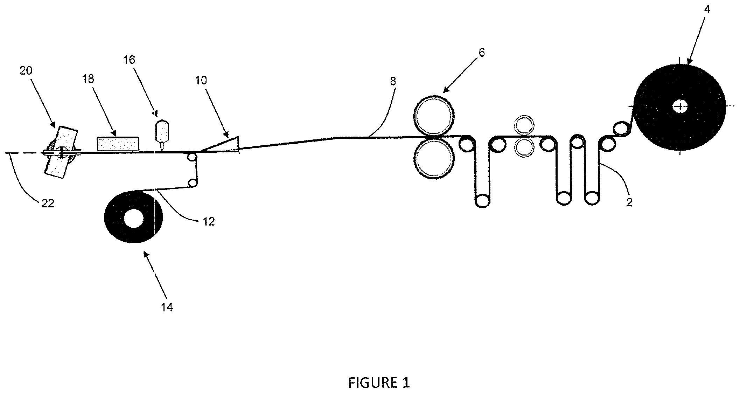

The apparatus shown in Figure 1 generally comprises: supply means for providing a continuous sheet of homogenised tobacco; crimping means for crimping the continuous sheet; rod forming means for gathering the continuous crimped sheet and circumscribing the gathered material with a wrapper to form a continuous rod; and cutting means for severing the continuous rod into a plurality of discrete rods. The apparatus also comprises transport means for transporting the continuous sheet of material downstream through the apparatus from the supply means to the rod forming means via the crimping means.

As shown in Figure 1, the supply means for providing a continuous sheet comprises a continuous sheet of homogenised tobacco 2 mounted on a bobbin 4. The crimping means comprises a pair of rotatable crimping rollers 6. In use, the continuous sheet of homogenised tobacco 2 is drawn from the first bobbin 4 and transported downstream to the pair of crimping rollers 6 by the transport mechanism via a series of guide and tensioning rollers. As the continuous sheet of homogenised tobacco 2 is fed between the pair of crimping rollers 6, the crimping rollers engage and crimp the sheet 2 to form a continuous crimped sheet of homogenised tobacco 8 having a plurality of spaced-apart ridges or corrugations substantially parallel to the longitudinal axis of the sheet through the apparatus.

The continuous crimped sheet of homogenised tobacco material 8 is transported downstream from the pair of crimping rollers 6 towards the rod forming means and fed through a converging funnel or horn 10. The converging funnel 10 gathers the continuous sheet of homogenised tobacco 8 transversely relative to its longitudinal axes. The sheet of material 8 assumes a substantially cylindrical configuration as it passes through the converging funnel 10.

Upon exiting the converging funnel 10, the gathered sheet of homogenised tobacco is wrapped in a continuous sheet of cigarette paper 12. The continuous sheet of paper is fed from a bobbin 14 and enveloped around the gathered continuous crimped sheet of homogenised tobacco material by an endless belt conveyor or garniture. As shown in Figure 1, the rod forming means comprises an adhesive application means 16 that applies adhesive to one of the longitudinal edges of the continuous sheet of paper, so that when the opposed longitudinal edges of the continuous sheet of paper are brought into contact they adhere to one other to form a continuous rod.

The rod forming means further comprises a drying means 18 downstream of the adhesive application means 16, which in use dries the adhesive applied to the seam of the continuous rod as the continuous rod is transported downstream from the rod forming means to the cutting means.

The cutting means comprises a rotary cutter 20 that severs the continuous rod into a plurality of discrete rods of unit rod length or multiple unit rod length.

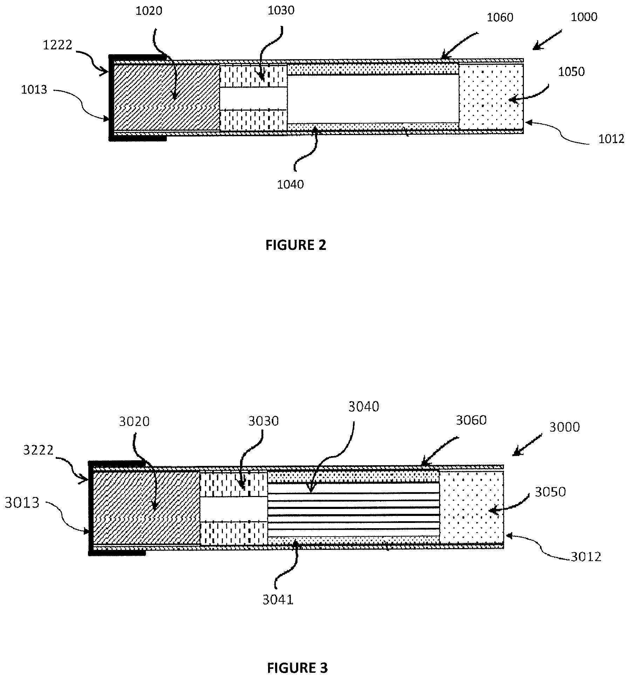

Figure 2 illustrates an embodiment of a heated aerosol-generating article 1000 comprising a rod as described herein. The article 1000 comprises four elements; an aerosol-forming substrate 1020, a hollow cellulose acetate tube 1030, a spacer element 1040, and a mouthpiece filter 1050. These four elements are arranged sequentially and in coaxial alignment and are assembled by a cigarette paper 1060 to form the aerosol-generating article 1000. The article 1000 has a mouth-end 1012, which a user inserts into his or her mouth during use, and a distal end 1013 located at the opposite end of the article to the mouth end 1012. The embodiment of an aerosol-generating article illustrated in Figure 2 is particularly suitable for use with an electrically-operated aerosol-generating device comprising a heater for heating the aerosol-forming substrate.

When assembled, the article 1000 is about 45 millimetres in length and has an outer diameter of about 7.2 millimetres and an inner diameter of about 6.9 millimetres.

The aerosol-forming substrate 1020 comprises a rod formed from a crimped and gathered sheet of homogenised tobacco wrapped in paper to form a plug. A distal end of the heated aerosol generating article is spanned by an aluminium foil 1222 which extends around the distal end and downstream along the article for about 10 mm. A user may inadvertently attempt to ignite the aerosol-forming substrate 1020 by applying a flame to the distal end 1013 and simultaneously drawing air through the mouthpiece. Should this occur, the aluminium foil spanning the distal end of the aerosol-generating article will swiftly spread the applied heat, thereby making it more difficult to increase the homogenised tobacco component to its ignition temperature. Furthermore, the foil substantially prevents air from being drawn into the article, thereby restricting the oxygen available in the region of the aerosol-forming substrate for combustion. This lowered propensity for ignition and combustion may be sufficient for the user to desist in the attempts to ignite the article.

An aerosol-generating article 1000 as illustrated in Figure 2 is designed to engage with an aerosol-generating device in order to be consumed. Such an aerosol-generating device includes means for heating the aerosol-forming substrate 1020 to a sufficient temperature to form an aerosol. Typically, the aerosol-generating device may comprise a heating element that surrounds the aerosol-generating article 1000 adjacent to the aerosol-forming substrate 1020, or a heating element that is inserted into the aerosol-forming substrate 1020.

Once engaged with an aerosol-generating device, a user draws on the mouth-end 1012 of the smoking article 1000 and the aerosol-forming substrate 1020 is heated to a temperature of about 375 degrees Celsius. At this temperature, volatile compounds are evolved from the sheet of cast-leaf tobacco of the aerosol-forming substrate 1020. These compounds condense to form an aerosol. The aerosol is drawn through the filter 1050 and into the user's mouth.

Figure 3 illustrates an alternative embodiment of a heated aerosol-generating article 3000 comprising a rod as described herein. The article 3000 comprises four elements; an aerosol-forming substrate 3020, a hollow cellulose acetate tube 3030, an aerosol-cooling element 3040, and a mouthpiece filter 3050. These four elements are arranged sequentially and in coaxial alignment and are assembled by a cigarette paper 3060 to form the aerosol-generating article 3000. The article 3000 has a mouth-end 3012, which a user inserts into his or her mouth during use, and a distal end 3013 located at the opposite end of the article to the mouth end 3012. The aerosol-cooling element 3040 acts as a spacer element as described in relation to Figure 2 as well as an aerosol-cooling element. In use, volatile substances released from the aerosol-forming substrate 3020 pass along the aerosol-cooling element 3040 towards a mouth end 3012 of the aerosol-generating article 3000. The volatile substances may cool within the aerosol-cooling element 3040 to form an aerosol that is inhaled by the user. In the embodiment illustrated in Figure 3, the aerosol-cooling element comprises a crimped and gathered sheet of polylactic acid circumscribed by a wrapper. As with the emodiment illustrated in Figure 2, a distal end of the heated aerosol generating article is spanned by an aluminium foil 3222 which extends around the distal end and downstream along the article for about 10 mm.

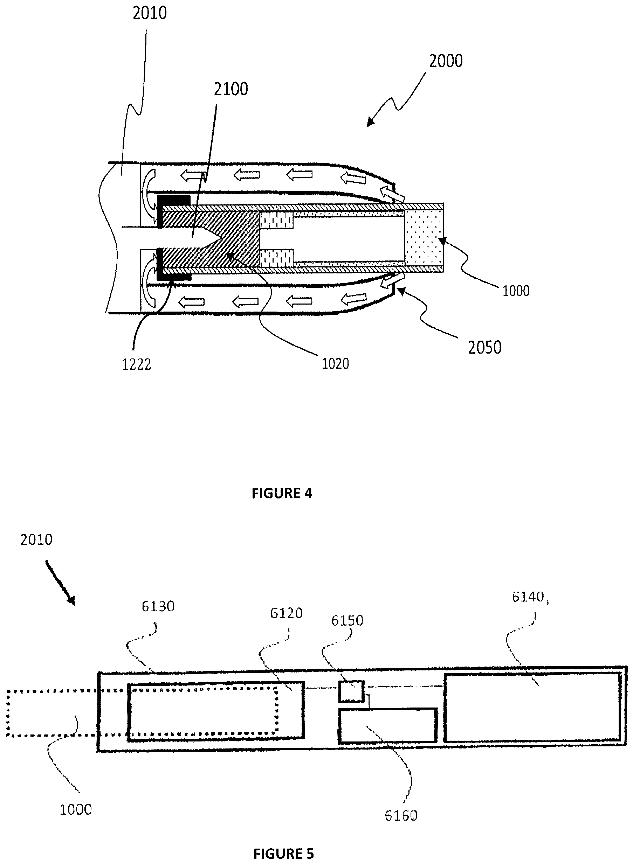

Figure 4 illustrates a portion of an electrically-operated aerosol-generating system 2000 that utilises a heating blade 2100 to heat an aerosol-generating substrate 1020 of an aerosol-generating article 1000. The heating blade is mounted within an aerosol article receiving chamber of an electrically-operated aerosol-generating device 2010. The aerosol-generating device defines a plurality of air holes 2050 for allowing air to flow to the aerosol-generating article 1000. On engagement with the aerosol-generating device 2010 the aluminium foil 1222 spanning the distal end 1013 is pierced by the heating blade 2100. Thus, when the heating blade is actuated and a user draws on the mouth end of the aerosol-generating article, air is able to flow into the article and deliver an aerosol to the user through the mouth end. Air flow is indicated by arrows on Figure 4. The aerosol-generating device comprises a power supply and electronics, which are illustrated in Figure 5. The aerosol-generating article 1000 of Figure 4 is as described in relation to Figure 2.

In Figure 5, the components of the aerosol-generating device 2010 are shown in a simplified manner. Particularly, the components of the aerosol-generating device 2010 are not drawn to scale in Figure 4. Components that are not relevant for the understanding of the embodiment have been omitted to simplify Figure 4.

As shown in Figure 5, the aerosol-generating device 2010 comprises a housing 6130. The heating element 6120 is mounted within an aerosol-generating article receiving chamber within the housing 6130. The aerosol-generating article 1000 (shown by dashed lines in Figure 5) is inserted into the aerosol-generating article receiving chamber within the housing 6130 of the aerosol-generating device 2010 such that the heating element 6120 is directly inserted into the aerosol-forming substrate 1020 of the aerosol-generating article 1000.

Within the housing 6130 there is an electrical energy supply 6140, for example a rechargeable lithium ion battery. A controller 6150 is connected to the heating element 6120, the electrical energy supply 6140, and a user interface 6160, for example a button or display. The controller 6150 controls the power supplied to the heating element 6120 in order to regulate its temperature.

The exemplary embodiments described above are not limiting. In view of the above-discussed exemplary embodiments, other embodiments consistent with the above exemplary embodiment will now be apparent to one of ordinary skill in the art.

## Claims

1. A heated aerosol-generating article (1000) for use with an aerosol-generating device comprising a heating element, the heated aerosol-generating article being in the form of a rod having a mouth end (1012) and a distal end (1013) upstream from the mouth end, the aerosol-generating article having an aerosol-forming substrate (1020) located upstream of the mouth end within the rod, in which the distal end of the heated aerosol-generating article is spanned by a non-flammable thermally-conductive material (1222) to spread heat away from the distal end of the rod and to prevent air flow into the distal end of the rod, thereby reducing the risk of inadvertent ignition of the rod by application of a flame, the non-flammable thermally-conductive material being rupturable to enable air-flow into the distal end of the rod.

2. A heated aerosol-generating article according to claim 1 in which the non-flammable thermally-conductive material is or comprises metal foil.

3. A heated aerosol-generating article according to claim 2 in which the metal foil is aluminium foil.

4. A heated aerosol-generating article according to claim 1, 2, or 3 in which the non-flammable thermally-conductive material spans the distal end of the rod and extends downstream along the rod to dissipate heat directly applied to the distal end of the rod.

5. A heated aerosol-generating article according to claim 4 in which the non-flammable thermally-conductive material extends downstream along the rod by at least 10 mm.

6. A heated aerosol-generating system comprising, a heated aerosol-generating article as defined in any of claims 1 to 5, and an aerosol-generating device having a heating element, the aerosol-generating device comprising a rupturing element for rupturing the thermally-conductive material spanning the distal end of the heated aerosol-generating article to allow air to be drawn through the aerosol-generating article when a user draws on the mouth end of the rod.

7. A heated aerosol-generating system according to claim 6 in which the rupturing element is a heating element arranged to be inserted into the distal end of the heated aerosol-generating article when the heated aerosol-generating article is engaged with the aerosol-generating device to rupture the non-flammable thermally-conductive material and heat the aerosol-forming substrate to form an aerosol.

8. A method of smoking a heated aerosol-generating article as defined in any of claims 1 to 5, the method comprising the steps of;
a) coupling the distal end of the rod with an aerosol-generating device having a heating element,
b) rupturing the non-flammable thermally-conductive material spanning the distal end of the rod,
c) actuating the heating element to heat the aerosol-forming substrate and generate an aerosol, and
d) inhaling the aerosol through the mouth end of the rod.

9. A method according to claim 8 in which the step of coupling the distal end of the rod with the aerosol-generating device causes a rupturing element to penetrate the distal end of the aerosol-generating article thereby rupturing the non-flammable thermally-conductive material.

10. A method according to claim 9 in which the rupturing element is the heating element of the aerosol-generating device.

## Patentansprüche

1. Beheizter aerosolerzeugender Artikel (1000) zum Gebrauch mit einer Aerosolerzeugungsvorrichtung, die ein Heizelement aufweist, wobei der beheizte aerosolerzeugende Artikel die Form eines Stocks mit einem Mundende (1012) und einem distalen Ende (1013) zuströmseitig von dem Mundende aufweist, der aerosolerzeugende Artikel ein aerosolbildendes Substrat (1020) aufweist, das zuströmseitig des Mundendes innerhalb des Stocks angeordnet ist, wobei sich über das distale Ende des beheizten aerosolerzeugenden Artikels ein nicht brennbares wärmeleitfähiges Material (1222) erstreckt, um Wärme von dem distalen Ende des Stocks abzuführen und Luftstrom in das distale Ende des Stocks zu verhindern und dadurch das Risiko eines unabsichtlichen Zündens des Stocks durch Anwenden einer Flamme zu reduzieren, und das nicht brennbare wärmeleitfähige Material zerreißbar ist, um Luftstrom in das distale Ende des Stocks zu ermöglichen.

2. Beheizter aerosolerzeugender Artikel nach Anspruch 1, wobei das nicht brennbare wärmeleitfähige Material Metallfolie ist oder aufweist.

3. Beheizter aerosolerzeugender Artikel nach Anspruch 2, wobei die Metallfolie Aluminiumfolie ist.

4. Beheizter aerosolerzeugender Artikel nach Anspruch 1, 2 oder 3, wobei sich das nicht brennbare wärmeleitfähige Material über das distale Ende des Stocks erstreckt und sich nachgeschaltet entlang dem Stock erstreckt, um auf das distale Ende des Stocks direkt angewandte Wärme abzuleiten.

5. Beheizter aerosolerzeugender Artikel nach Anspruch 4, wobei sich das nicht brennbare wärmeleitfähige Material um mindestens 10 mm nachgeschaltet entlang dem Stock erstreckt.

6. Beheiztes Aerosolerzeugungssystem, aufweisend einen beheizten aerosolerzeugenden Artikel nach einem der Ansprüche 1 bis 5 und
eine Aerosolerzeugungsvorrichtung mit einem Heizelement, wobei die Aerosolerzeugungsvorrichtung ein Zerreißelement zum Zerreißen des wärmeleitfähigen Materials, das sich über das distale Ende des beheizten aerosolerzeugenden Artikels erstreckt, aufweist, um zu ermöglichen, dass Luft durch den aerosolerzeugenden Artikel gezogen werden kann, wenn ein Benutzer am Mundende des Stocks zieht.

7. Beheiztes Aerosolerzeugungssystem nach Anspruch 6, wobei das Zerreißelement ein Heizelement ist, das derart ausgeführt ist, dass es in das distale Ende des beheizten aerosolerzeugenden Artikels eingesetzt wird, wenn der beheizte aerosolerzeugende Artikel in Eingriff mit der Aerosolerzeugungsvorrichtung ist, um das nicht brennbare wärmeleitfähige Material zu zerreißen und das aerosolbildende Substrat zu erwärmen, um ein Aerosol zu bilden.

8. Verfahren zum Rauchen eines beheizten aerosolerzeugenden Artikels nach einem der Ansprüche 1 bis 5, wobei das Verfahren die Schritte aufweist;
a) Koppeln des distalen Endes des Stocks mit einer Aerosolerzeugungsvorrichtung mit einem Heizelement,
b) Zerreißen des nicht brennbaren wärmeleitfähigen Materials, das sich über das distale Ende des Stocks erstreckt,
c) Betätigen des Heizelements, um das aerosolbildende Substrat zu erwärmen und ein Aerosol zu erzeugen, und
d) Einatmen des Aerosols durch das Mundende des Stocks.

9. Verfahren nach Anspruch 8, wobei der Schritt des Koppelns des distalen Endes des Stocks mit der Aerosolerzeugungsvorrichtung bewirkt, dass ein Zerreißelement das distale Ende des aerosolerzeugenden Artikels durchdringt und dadurch das nicht brennbare wärmeleitfähige Material zerreißt.

10. Verfahren nach Anspruch 9, wobei das Zerreißelement das Heizelement der Aerosolerzeugungsvorrichtung ist.

## Revendications

1. Article de génération d'aérosol chauffé (1000) destiné à être utilisé avec un dispositif de génération d'aérosol comprenant un élément de chauffage, l'article de génération d'aérosol chauffé étant sous la forme d'une tige ayant une extrémité buccale (1012) et une extrémité distale (1013) en amont de l'extrémité buccale, l'article de génération d'aérosol ayant un substrat formant aérosol (1020) situé en amont de l'extrémité buccale à l'intérieure de la tige, où l'extrémité distale de l'article de génération d'aérosol chauffé est recouverte d'un matériau thermoconducteur non inflammable (1222) pour écarter la chaleur loin de l'extrémité distale de la tige pour empêcher l'écoulement d'air dans l'extrémité distale de la tige, en réduisant ainsi le risque d'allumage par inadvertance de la tige par l'application d'une flamme, le matériau thermoconducteur non inflammable pouvant être rompu pour permettre l'écoulement d'air dans l'extrémité distale de la tige.

2. Article de génération d'aérosol chauffé selon la revendication 1, dans lequel le matériau thermoconducteur non inflammable est ou comprend une feuille métallique.

3. Article de génération d'aérosol chauffé selon la revendication 2 dans lequel la feuille métallique est une feuille d'aluminium.

4. Article de génération d'aérosol chauffé selon la revendication 1, 2 ou 3, dans lequel le matériau thermoconducteur non inflammable recouvre l'extrémité distale de la tige et s'étend en aval le long de la tige pour dissiper la chaleur directement appliquée à l'extrémité distale de la tige.

5. Article de génération d'aérosol chauffé selon la revendication 4, dans lequel le matériau thermoconducteur non inflammable s'étend en aval le long de la tige d'au moins 10 mm.

6. Système de génération d'aérosol chauffé comprenant un article de génération d'aérosol chauffé tel que défini dans l'une quelconque des revendications 1 à 5, et
un dispositif de génération d'aérosol ayant un élément de chauffage, le dispositif de génération d'aérosol comprenant un élément de rupture pour la rupture du matériau thermoconducteur couvrant l'extrémité distale de l'article de génération d'aérosol chauffé pour permettre l'extraction d'air via l'article de génération d'aérosol lorsqu'un utilisateur tire sur l'extrémité buccale de la tige.

7. Système de génération d'aérosol chauffé selon la revendication 6, dans lequel l'élément de rupture est un élément de chauffage disposé à être inséré dans l'extrémité distale de l'article de génération d'aérosol chauffé lorsque l'article de génération d'aérosol chauffé est engagé avec le dispositif de génération d'aérosol pour rompre le matériau thermoconducteur non inflammable et pour chauffer le substrat formant aérosol pour former un aérosol.

8. Procédé de fumage d'un article de génération d'aérosol chauffé selon l'une quelconque des revendications 1 à 5, le procédé comprenant les étapes de :
a) l'accouplement l'extrémité distale de la tige avec un dispositif de génération d'aérosol ayant un élément de chauffage,
b) la rupture du matériau thermoconducteur non inflammable recouvrant l'extrémité distale de la tige,
c) l'actionnement de l'élément de chauffage pour chauffer le substrat formant aérosol et générer un aérosol, et
d) l'inhalation de l'aérosol par l'extrémité buccale de la tige.

9. Procédé selon la revendication 8, dans lequel l'étape de couplage de l'extrémité distale de la tige avec le dispositif de génération d'aérosol provoque qu'un élément de rupture pénètre l'extrémité distale de l'article de génération d'aérosol, ce qui rompt ainsi le matériau thermoconducteur non inflammable.

10. Procédé selon la revendication 9, dans lequel l'élément de rupture est l'élément de chauffage du dispositif de génération d'aérosol.
